# FASCICULE DE BREVET EUROPEEN

(11) **EP 0 503 019 B1**
(45) Date de publication et mention de la délivrance du brevet: **27.03.1996**
(21) Numéro de dépôt: 91916039.0
(22) Date de dépôt: 30.09.1991
(51) Int. Cl.: A61F 5/56

(54) **APPAREIL PREVENTIF DU RONFLEMENT**
VORRICHTUNG ZUM VERHINDERN DES SCHNARCHENS
SNORING PREVENTION DEVICE

(30) Priorité: 03.10.1990 CH 3175/90
(43) Date de publication de la demande: 16.09.1992
(73) Titulaire: TRUFFER, Ernest, CH-3960 Sierre (CH)
(72) Inventeur: TRUFFER, Ernest, CH-3960 Sierre (CH)
(74) Mandataire: Ganguillet, Cyril
(86) Numéro de dépôt international: CH9100204
(87) Numéro de publication internationale: WO9205752

(56) Documents cités:
- EP-A- 0 264 516
- EP-A- 0 312 368
- US-A- 4 715 368
- US-A- 4 901 737

## Description

La présente invention se rapporte à un appareil préventif du ronflement.

On distingue deux types de ronflement, en fonction de la localisation de leur origine. Le premier type de ronflement, le ronflement vélaire, est produit par la vibration de l'ensemble des structures du palais mou, soit le voile du palais, les piliers amygdaliens antérieurs et postérieurs et la luette. Le ronflement vélaire découle d'une vibration du palais mou créée par les flux d'air inspiratoires, nasal et oral, qui font flotter le palais mou comme un drapeau. L'intensité sonore de ces vibrations est accentuée par l'ouverture de la cavité buccale qui agit comme une caisse de résonance.

Le deuxième type, soit le ronflement pharyngé, est une sorte de râle, de râlement, voire de cornage. Il est provoqué par l'obstruction partielle de la filière pharyngée par la base de la langue avec, par moments, son occlusion totale par accollement, selon les lois de la dynamique des fluides, de cette base de langue contre la paroi postérieure du pharynx, provoquant ainsi les arrêts respiratoires (apnées) constituant le Sleep Apnea Syndrom (SAS). Il s'agit ici d'apnée obstructive, par opposition à l'apnée centrale qui est d'origine cérébrale.

Il convient de préciser que les deux formes de ronflement décrites ci-dessus peuvent parfaitement être cumulées chez le même individu.

Le ronflement, qui est toujours une gêne pour l'entourage, n'est pas inoffensif pour le ronfleur lui-même, essentiellement dans le cas du ronflement pharyngé accompagné d'apnées obstructives.

Il existe, depuis quelques années, des techniques chirurgicales tendant à corriger ces ronflements. Cependant, la chirurgie maxilaire que nécessite le ronflement pharyngé est une chirurgie lourde, l'opération durant plusieurs heures et l'uvulo-pharyngo-palato-plastie (UPPP) corrigeant le ronflement vélaire n'est pas sans inconvénients. Cela explique la faveur des prothèses et autres appareils préventifs.

L'appareil proposé dans le brevet US-A-4,669,459 vise tout spécialement le ronflement vélaire et met en jeu une pastille destinée à appuyer sur le palais mou, de manière à empêcher celui-ci de se mettre en vibration. L'appareil est fixé au moyen de crochets dentaires. Il n'a, en principe, pas d'action sur le ronflement pharyngé.

Certains appareils s'attaquent au ronflement pharyngé et, partant de la même constatation que ce type de ronflement est associé à un rétrognatisme de la mâchoire inférieure, ils tendent à provoquer un avancement de la mâchoire inférieure pour écarter la base de la langue de la paroi pharyngée postérieure et ainsi élargir la filière laryngée. Ces appareils ont des formes qui ressemblent à des protège-dents, tels qu'en utilisent les sportifs, les boxeurs en particulier. A l'intérieur de cette famille d'appareils, on peut remarquer que la plupart d'entre eux offrent une sorte de canal respiratoire, un peu comme l'embout d'un tuba de plongée.

Tel est le cas par exemple du dispositif illustré dans le document US-A-4,715,368 qui comporte une partie médiane pourvue de crochets dentaires et une partie inférieure appuyant sur l'arc dentaire inférieur en projetant celui-ci; un canal respiratoire étant aménagé dans la partie médiane.

Un résultat comparable est obtenu avec le dispositif selon le document US-A-4,901,737, le passage respiratoire étant ici créé par l'épaisseur de la partie médiane et l'écartement qu'elle impose entre les deux arcs dentaires, la bouche étant maintenue en position semi-ouverte.

Les appareils tels que ceux illustrés dans les documents US 4,715,368 et US 4,901,737 permettent sans doute de combattre le ronflement pharyngé, mais pas le ronflement vélaire.

D'autres appareils encore, s'attachent à tirer la base de la langue vers l'avant, non pas en agissant sur la mâchoire inférieure, mais en agissant sur la langue directement. Ainsi, dans le brevet US 3,132,647, une sorte de cuillière appuie directement sur la langue. Enfin, dans le brevet US 4,304,227, la langue s'introduit dans un logement et y reste confinée et tirée en avant par succion, un peu comme font les enfants en introduisant la langue dans le goulot d'une bouteille.

En résumé, les appareils déjà connus ne peuvent combattre que l'un des deux types de ronflement décrits plus haut. Certains d'entre eux ne paraissent pas être particulièrement agréables à porter. Il n'est pas nécessaire de s'attarder sur les défauts des appareils connus, chacun pouvant parfaitement s'imaginer la sensation que créent ces appareils lorsqu'on les porte. Sans doute dans certains cas le ronflement est-il vaincu par la disparition du sommeil.

On relèvera encore qu'aucun appareil connu n'est à même de garder la bouche du ronfleur fermée, même si dans le dernier brevet cité, la respiration orale est exclue ou déclarée telle.

La présente invention a pour but de proposer un appareil capable d'éliminer les deux types de ronflement, soit isolement, soit simultanément, étant précisé que cet appareil suppose que la respiration par voie nasale est possible. En d'autres termes l'appareil selon l'invention ne vise nullement le cas où le ronfleur a le nez bouché.

La définition de l'appareil préventif du ronflement selon l'invention est donnée dans la revendication 1. Des formes d'exécution sont définies dans les revendications subordonnées à la revendication 1.

On décrit ci-après à titre d'exemple un appareil selon l'invention en se référant au dessin où:
- la figure 1: montre une coupe sagittale de la région buccopharyngée et sert de support à l'explication des deux types de ronflement;
- la figure 2: montre une vue de face de l'appareil selon l'invention;
- la figure 3: montre une vue de profil de l'appareil selon l'invention;
- la figure 4: montre l'appareil selon l'invention, vu de l'arrière; et
- la figure 5: présente une coupe similaire à celle de la figure 1, mais dans la configuration que provoque l'appareil une fois mis en place;
En observant la figure 1, on distingue le palais osseux 1, prolongé par le voile du palais et la luette 2. On voit également la paroi pharyngée 3 et la base de la langue 4, qui décrivent entre elles un espace, la filière pharyngée 5.

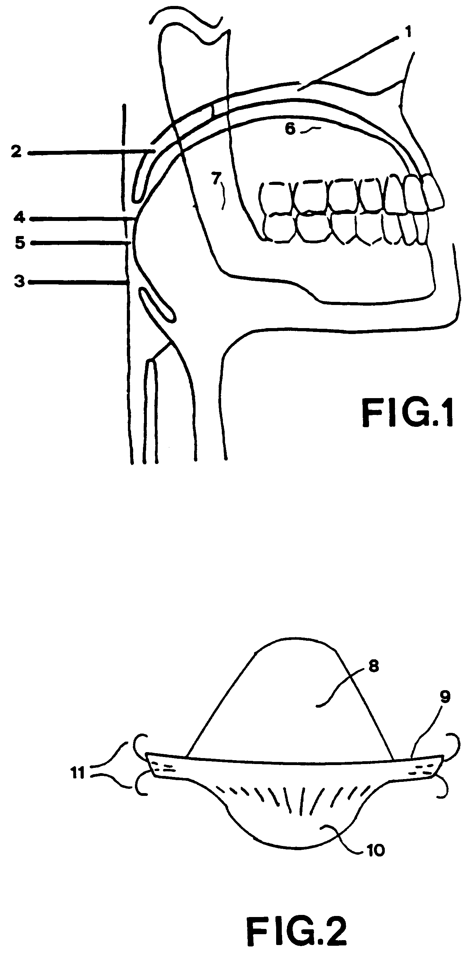

La configuration illustrée à la figure 1, permet de décrire les deux types de ronflement. Le ronflement vélaire est produit par l'écoulement des deux flux d'air inspiratoire. Le flux nasal s'écoule sur la surface supérieure du voile et de la luette 2. Le flux buccal circule entre la surface inférieure du palais et la partie supérieure 6 de la langue. Ces deux flux, arrivant de part et d'autre de la luette et du voile 2, mettent ceux-ci en vibration, ce qui provoque des ondes sonores qui créent le ronflement. La cage de résonance que constitue la cavité buccale amplifie ces ondes sonores et les rend plus bruyantes.

Le ronflement pharyngé est provoqué par l'étroitesse de la filière pharyngée 5. En effet, le passage de l'air dans un conduit aussi étroit provoque un râle, voire un cornage. Enfin l'accollement complet de la base de la langue 4 et de la paroi pharyngée 3 provoque l'apnée obstructive.

Bien que l'essentiel de l'appareil soit en fait réalisé d'une seule pièce et dans la même matière, un plastique dur, il convient de distinguer trois parties dont chacune a des fonctions propres. En se référant aux figures 2 à 4, on distingue donc tout d'abord la partie supérieure 8 de l'appareil. Cette partie est destinée à prendre appui sur le palais osseux et à assurer le positionnement en bouche de l'appareil. A cette fin, elle est confectionnée d'après l'empreinte du palais et réalisée en plastique dur. La partie supérieure 8 de l'appareil épouse ainsi parfaitement le palais.

La deuxième partie est la partie médiane d'appui 9 de l'appareil. Cette partie médiane 9 est, comme on l'a dit, solidaire des deux autres parties. Elle est conformée en un rebord latéral saillant sur lequel les arcs dentaires supérieur et inférieur viennent se positionner. D'où la nécessité de la prise d'empreinte préalable des deux arcs dentaires pour modeler la partie médiane d'appui 9 en fonction d'une part des caractéristiques propres de la personne qui portera l'appareil et d'autre part du degré d'avancement recherché de la mâchoire inférieure qui, une fois déterminé, vaudra aussi par définition pour la troisième partie de l'appareil. Il convient à ce stade de la description de souligner que, contrairement à la plupart des appareils connus, l'appareil selon l'invention ne propose pas de loger les arcs dentaires dans des rigoles ou gouttières en forme de U. Il n'y a donc dans l'appareil selon l'invention aucun élément s'intercalant entre les dents et les lèvres.

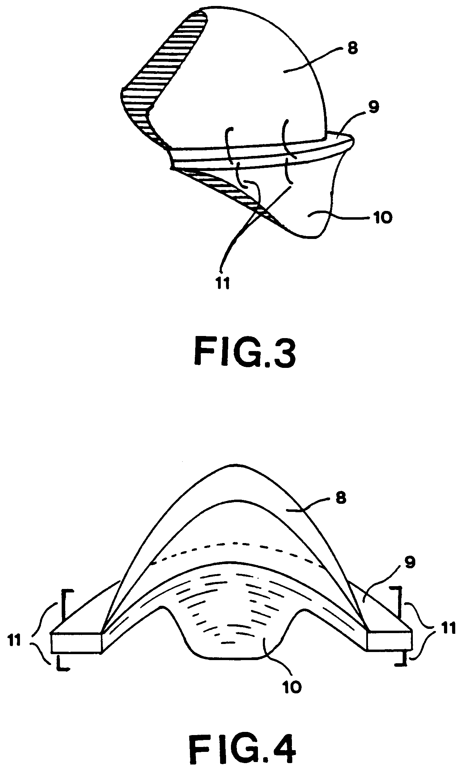

Une troisième partie ou partie antéro-inférieure 10 soutient la mâchoire inférieure vers l'avant en se plaçant derrière les dents antérieures de la mâchoire inférieure. On le voit d'ailleurs mieux sur la figure 4. Il est important de relever que la force nécessaire à projeter la mâchoire inférieure vers l'avant n'est pas transmise à celle-ci par les seules dents antérieures de l'arc inférieur. En effet, l'ensemble de la dentition se partage l'effort et c'est l'une des particularités originales de l'appareil. En effet, si les arcs dentaires sont maintenus en contact étroit avec leur empreinte respective, chaque dent offre une résistance au glissement et au retour à la configuration naturelle telle qu'illustrée à la figure 1. Certes, en serrant simplement les mâchoires l'une contre l'autre, le porteur peut réaliser cet effet. Mais il ne le réalisera certainement pas une fois pris de sommeil. L'appareil selon l'invention permet précisément la réalisation continue de cet effet en assurant par lui même la conservation de la bouche dans la position fermée. L'avantage de cette situation est, comme on l'a précisé d'entrée de cause, que la respiration orale est en fait supprimée. Le maintien de la bouche en position fermée est rendu possible par la mise en jeu de crochets dentaires 11. Le nombre, la forme et la disposition des crochets dépendent de l'état de la bouche du porteur d'appareil. Pour ce qui concerne la réalisation des crochets, dans tous leurs détails, on s'en remet à la technique usuelle des mécaniciens-dentistes. Il convient de souligner que l'utilisation de crochets a été proposée dans l'un des brevets constituant l'art antérieur, mais on notera que leur fonction n'est nullement de produire la fermeture de la bouche; ils ne s'appliquent d'ailleurs qu'à la mâchoire supérieure, ce qui interdit évidemment l'effet recherché dans la présente invention. On notera aussi que l'utilisation de gouttières en U, dont on a déjà parlé, exclut de facto l'utilisation de crochets.

La figure 3 permet de prendre mieux conscience des formes et volumes des différentes parties de l'appareil et permet de comprendre que la force de retrait exercée par la mâchoire inférieure ne s'exprime pas seulement dans une direction avant-arrière mais est transformée partiellement en une composante verticale qui appuie sur le palais dur par l'intermédiaire de la partie supérieure 8.

La figure 4 montre l'appareil vu de l'arrière et permet de constater que la langue est libre dans la bouche et dispose d'un espace sensiblement égal à son espace naturel.

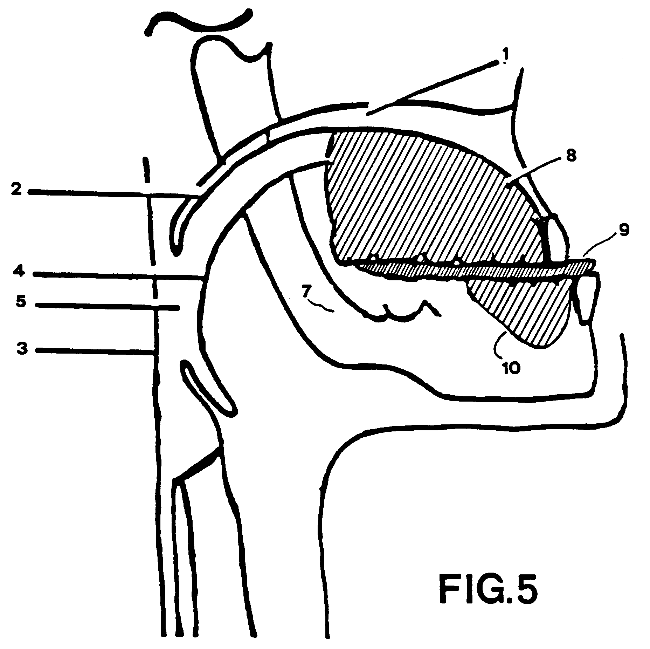

La figure 5 permet, de constater les modifications qu'entraîne le port de l'appareil selon l'invention. On remarque en premier lieu que la mâchoire inférieure 7 est subluxée et projetée vers l'avant. Il en résulte que la base de la langue 4 est également projetée vers l'avant et se distance ainsi de la paroi pharyngée 3, entraînant un élargissement de la filière pharyngée 5, qui est proportionnel à cette projection. On remarque que la luette 2 est également mieux dégagée.

Le ronflement pharyngé est ainsi éliminé, la filière pharyngée retrouvant une taille suffisante pour supprimer le râle et le risque d'apnée.

Le ronflement vélaire est également éliminé car, la bouche étant maintenue close par l'application des dents supérieures et inférieures sur la partie médiane, qui sont bloquées dans cette position par les crochets, il n'y a plus de flux inspiratoire oral et par conséquent pas de mise en vibration du palais mou non plus. Le fait que la bouche est close et maintenue telle a pour seconde conséquence que celle-ci ne peut plus fonctionner comme cage de résonance. Enfin, s'il persistait quelques vibrations résiduelles, leur sonorité serait étouffée par l'occlusion buccale.

L'appareil selon l'invention suppose bien sûr la réalisation de trois conditions, à savoir la possibilité de respirer par le nez, l'existence d'un nombre suffisant de dents pour pouvoir fixer l'appareil en place et finalement une laxité suffisante de l'articulation de la mâchoire. Cette laxité s'apprécie par la capacité et l'étendue du déplacement, dans le sens antéro-postérieur, de la mâchoire inférieure.

Etant donné la spécificité de la morphologie maxillo-dentaire de chaque individu, il n'est pas possible d'obtenir un appareil standard. La confection de l'appareil nécessite la prise des empreintes dentaires et, par conséquent, la collaboration avec le médecin-dentiste qui procèdera comme pour une prothèse dentaire. Une fois l'appareil réalisé selon ses indications et en application de la technique prosthétique dentaire, l'intéressé pourra le placer en bouche et l'enlever, comme cela se fait pour n'importe quelle prothèse dentaire amovible.

Les tests effectués montrent que l'appareil, après la période initiale d'adaptation, est en général bien supporté et ne constitue qu'une gêne facilement surmontée. Les expériences d'utilisation, sur une durée atteignant cinq ans pour certains, n'ont pas conduit à constater une détérioration de l'émail des dents ni de troubles de l'articulé dentaire, ni de l'implantation des dents ni de l'articulation temporo-maxillaire.

Les conditions nécessaires d'utilisation étant prises en compte, les avantages de l'appareil selon l'invention sont pour l'essentiel qu'il supprime totalement les deux types de ronflement, et cela simultanément, le cas échéant. D'autre part, l'appareil est conformé de manière à avoir une présence en bouche la plus discrète possible grâce au fait que le nécessaire intervalle entre les arcs dentaires est réduit au strict minimum que représente la partie médiane. Cette caractéristique résulte en premier lieu de ce que les arcs dentaires ne sont pas logés dans des gouttières en U et en second lieu de ce qu'il ne comporte pas de canal respiratoire. La réalisation de l'appareil fait certes appel à des spécialistes de l'art dentaire, ce qui influe sur son prix. Cependant, l'appareil met en jeu des techniques dentaires bien connues et maîtrisées qui garantissent un résultat parfaitement adapté à la personne concernée et n'est pas plus gênant que n'importe quelle autre prothèse dentaire. Enfin, l'appareil permet de supprimer le grincement des dents.

## Revendications

1. Appareil préventif du ronflement comportant une partie médiane (9), en forme de rebord latéral saillant, destinée à servir d'appui aux deux arcs dentaires, partie médiane sur laquelle sont fixés des crochets dentaires (11) collaborant pour les uns avec l'arc supérieur et pour les autres avec l'arc inférieur; l'appareil comportant également une partie antéro-inférieure (10) épousant la forme de la mâchoire inférieure, la face inférieure de la partie médiane et/ou la partie antéro-inférieure étant décallées vers l'avant par rapport à la position relative de l'arc dentaire supérieur tel que déterminé par la face supérieure de la partie médiane pour projeter la mâchoire inférieure vers l'avant, caractérisé en ce qu'il comporte une partie supérieure (8) réalisée d'après une empreinte du palais, laquelle est solidaire de la partie médiane et est positionnée par rapport à celle-ci de manière à s'appuyer sur le palais; l'épaisseur de la partie médiane étant limitée à la quantité de matière nécessaire à assurer la rigidité de l'appareil; l'appareil, une fois fixé en bouche au moyen des crochets, formant avec les deux arcs dentaires un tout solidaire dépourvu d'orifice de taille suffisante à permettre la respiration bucale.

2. Appareil selon la revendication 1, caractérisé en ce qu'il est réalisé en matière plastique dure d'après une empreinte de l'un au moins des arcs dentaires.

## Claims

1. A snoring prevention device comprising a median part (9) in the shape of a lateral protruding edge, intended to serve as a support for both dental arches, median part on which are attached dental hooks (11) cooperating for the ones with the upper arch and for the others with the lower arch; said device also comprising an antero-inferior part (10) fitting the shape of the lower jaw, the lower face of the median part and/or the antero-inferior part being shifted forward, compared to the relative position of the upper dental arch given by the upper face of the median part, to project the lower jaw forward, characterised in that is comprises an upper part (8) made according to an impression of the palate, and which is integral with the median part and which is positioned in comparison with the latter so as to rest on the palate; the thickness of the median part being limited to the quantity of material necessary to ensure the rigidity of the device; the device, when fixed in the mouth by means of the hooks, forming an integral unit with the two dental arches, without openings having dimensions that would be sufficient to allow the mouth breathing.

2. Device according to claim 1, characterised in that it is made from hard plastic material according to an impression of at least one of the dental arches.

## Patentansprüche

1. Gerät zur Verhinderung des Schnarchens, mit einem mittleren Teil (9) in Form eines seitlich vorspringenden Randes, der als Stütze für die beiden Zahnreihen dient und an dem Zahnhaken (11) befestigt sind, die zum einen mit der oberen Zahnreihe und zum anderen mit der unteren Zahnreihe zusammenwirken; das Gerät weist außerdem ein vorderes unteres Teil (10) auf, das an die Form des Unterkiefers anpasst ist, wobei die untere Fläche des mittleren Teils und/oder das vordere untere Teil nach vorn verschoben ist, relativ zur oberen Zahnreihe und bestimmt ist durch die obere Fläche des mittleren Teils, um dergestalt den Unterkiefer nach vorne zu verschieben, dadurch gekennzeichnet, daß es ein drittes oberes Teil (8) aufweist, das nach einem Gaumenabdruck angefertigt ist, wobei es fest mit dem mittleren Teil verbunden ist und derart bezüglich des letzteren angeordnet ist, daß er sich auf dem Gaumen abstützt; die Dicke des mittleren Teils ist begrenzt auf die zur erforderlichen Steifigkeit des Gerätes erforderliche Materialmenge; das Gerät bildet nach der Befestigung im Mund mittels der Haken zusammen mit den beiden Zahnreihen eine feste Gesamtanordnung ohne Öffnung von ausreichender Größe, um eine Mundatmung zu ermöglichen.

2. Gerät nach Anspruch 1, dadurch gekennzeichnet, daß es nach dem Abdruck wenigstens einer der Zahnreihen aus Hartkunststoff gefertigt wird.
